# EUROPÄISCHE PATENTSCHRIFT

(11) **EP 1 295 590 B1**
(45) Veröffentlichungstag und Bekanntmachung des Hinweises auf die Patenterteilung: **03.01.2007**
(21) Anmeldenummer: 02015153.6
(22) Anmeldetag: 06.07.2002
(51) Int. Cl.: A61K 8/362, A61K 8/41, A61Q 5/10, A61Q 5/04, C07C 51/41

(54) **Zweikomponenten-Mittel mit zeitlichem pH-Gradienten und Verfahren zur Behandlung von Haaren**
Two-component agent with a temporary pH gradient and process for treating hair
Agent à deux composants avec un gradient de pH temporaire et procédé pour traiter la chevelure

(30) Priorität: 20.09.2001 DE 10146350
(43) Veröffentlichungstag der Anmeldung: 26.03.2003
(73) Patentinhaber: Wella Aktiengesellschaft, 64274 Darmstadt (DE)
(72) Erfinder: Kripp, Thomas, Dr., 64407 Fränkisch-Crumbach (DE); Grasser, Beate, 65795 Hattersheim (DE); Koschik, Achim, 64297 Darmstadt (DE)

(56) Entgegenhaltungen:
- DE-A- 19 860 239
- DE-A- 19 902 246
- US-A- 5 132 107

## Beschreibung

Verfahren zur Behandlung von Haaren, insbesondere zur Pflege oder zur dauerhaften Verformung von Haaren, unter Verwendung eines Zweikomponenten-Mittels mit einem sich nach dem Vermischen der Komponenten verzögerten, innerhalb von 1 bis 60 Minuten selbsttätig verändernden pH-Wert, enthaltend in einer Komponente (A) mindestens eine Säure und in einer anderen Komponente (B) mindestens eine Base, wobei die Säure und/oder die Base eine geringe Wasserlöslichkeit aufweisen.

Wenngleich die meisten chemischen, technischen, lebensmitteltechnologischen und kosmetischen Produkte von Beginn bis Ende der Anwendung den gleichen pH-Wert haben (sollen), gab es für spezielle Anwendungen immer wieder Versuche, Systeme zu schaffen, die nach der Applikation ihren pH selbstständig ändern.

Für diese Anforderung können verschiedene Gründe vorliegen, beispielsweise:
- Eine Substanz ist bei dem pH, bei dem sie ihre Wirkung am besten entfalten kann, nur begrenzt lagerstabil;
- eine pH-abhängige Wirkung soll in ihrer Intensität zeitabhängig gesteuert werden;
- zwei pH-abhängige Wirkungen mit unterschiedlichem pH-Optimum sollen, trotz nur einmaliger Auftragung, nacheinander ablaufen;
- eine Reaktionsverzögerung soll erreicht werden, um eine vorherige Diffusion zu ermöglichen;
- Ein Farbumschlag soll das Ende einer Einwirkzeit anzeigen.

Aus der eigenen DE 1089124 A1 ist ein Verfahren zur Dauerverformung bekannt, bei dem man mit Hilfe von Urease aus Harnstoff allmählich Ammoniak freisetzt und so für einen Anstieg des pH während der Einwirkungszeit sorgt.

Aus der eigenen DE 2349050 A ist ein Mittel und ein Verfahren bekannt, bei dem die Wellaktivität über das Absenken des Anfangs-pH-Wertes kontinuierlich verringert wird. Dem Verformungsmittel werden kurz vor dem Gebrauch solche alkalisch spaltbaren organischen Verbindungen mit Ester- oder Halogengruppen im Molekül zugesetzt, die imstande sind, bei der Spaltung Säure entstehen zu lassen. Dadurch soll die Konzentration an Alkali während der Einwirkungszeit allmählich und in dem gewünschten Umfang abnehmen. Als geeignete Ester sind u. a. Essigsäureethylester und Triacetin genannt. Bei den Estergruppen enthaltenden Verbindungen kann eine Beschleunigung der Spaltung durch zusätzliche Beigabe der Lipase Pankreatin erreicht werden.

Aus der eigenen DE-PS 19860239 C2 ist ein System bekannt, bei dem durch enzymatische Spaltung von Estern, insbesondere z. B. Triglyceriden, und gleichzeitigem Gegenpuffern der pH-Verlauf einer Dauerwelllösung zeitabhängig absinkt, um die anfänglich hohe Wellaktivität erst nach einiger Zeit abzusenken und zudem ein Unterschreiten von pH 7,0 zu verhindern.

Aus der eigenen DE 19902246 A1 ist ein System bekannt, bei dem durch nichtenzymatische Hydrolyse eines hinreichend instabilen Lactons eine pH-Absenkung erfolgt, deren Geschwindigkeit und End-pH bei gegebenen Rahmenbedingungen (Temperatur, Anfangs-pH etc.) von Art und Konzentration des betreffenden Eduktes abhängt.

Jedes dieser Systeme hat jedoch gravierende Nachteile: Wird der pH-Gradient durch enzymkatalysierte Reaktionen erzeugt, so ist die Einstellung (und vor allem die Aufrechterhaltung) eines definierten Verlaufes äußerst schwierig. Unter Lagerbedingungen verändert sich üblicherweise die Enzymaktivität und somit die Kinetik der pH-Änderung. Zudem wird in der Regel eine Drei-Komponenten-Verpackung notwendig sein: Eine für das Hauptprodukt, eine für das (streng wasserfreie) Hydrolyse-Objekt sowie eine für das Enzym. Zusätzlich entsteht bei der Applikation ein Mehraufwand bezüglich des (homogenen, vollständigen!) Mischens der drei Komponenten; auch ist es nicht unproblematisch, die darauf folgende Anwendung hinreireichend schnell durchzuführen.

Die in der DE 19902246 A1 genutzte Spontanhydrolyse eines Lactons umgeht zwar die Problematik der Enzymkatalyse; allerdings existiert nur eine recht begrenzte Zahl geeigneter Verbindungen. Das Hydrolyseverhalten des jeweiligen Stoffes ist dabei starr in der Natur der Substanz verankert und formulierungstechnisch kaum beeinflussbar, so dass keine nennenswerte Möglichkeit besteht, ein derartiges System an verschiedene Aufgaben oder Gegebenheiten anzupassen.

Allen hier geschilderten Lösungsansätzen ist zudem gemein, dass kurz vor der Applikation die _{"}normale" Produktmasse mit _{"}unnormalen" Komponenten (wasserfreien Suspensionen, kristallinen oder pulverförmigen Substanzen) versetzt werden müssen (der Ausdruck "normal" bezieht sich hier sowohl auch die chemische Zusammensetzung als auch auf die Anmutung: Farbe, Geruch, Fließverhalten, Viskosität, Haptik).

Es bestand daher die Aufgabe, ein Verfahren unter Verwendung eines Systems zu entwickeln, das einen zeitlichen pH-Gradienten aufbaut mit folgenden Eigenschaften:
- es enthält nur Komponenten mit _{"}normaler" Anmutung;
- es enthält keine hydrolytisch freizusetzenden Substanzen;
- es enthält keine enzymatisch freizusetzenden Substanzen;
- es weist eine hohe Lagerstabilität aller Komponenten auf;
- es benötigt maximal zwei getrennte Komponenten;
- es ist einstellbar bezüglich der Lage der durchlaufenen pH-Bereiche;
- es ist einstellbar bezüglich der Größe der durchlaufenen pH-Bereiche;
- es ist einstellbar bezüglich Richtung der pH-Änderung;
- es ist einstellbar bezüglich Geschwindigkeit der pH-Änderung;

Die Aufgabe wird gelöst durch den Gegenstand nach Anspruch 1.

Bei dem erfindungsgemäßen Verfahren zur Haarbehandlung wird eine Säure mit einer Base in Kontakt gebracht. Die zur Lösung der Aufgabe geeignete Säure und/oder die Base ist - im Gegensatz zu ihrem Salz - nur wenig wasserlöslich. Bevorzugt ist nur die Säure wenig wasserlöslich. Die Salzbildung (z. B. die Bindung der anfangs im Gemisch überschüssigen Base) erfolgt dann hauptsächlich mit jeweils dem kleinen Anteil der Säure, der sich entsprechend der Gleichgewichtskonstanten in Lösung befindet. Aufgrund der geringen Wasserlöslichkeit der Säure erfolgt die Salzbildung (und somit die Neutralisation der Base) verzögert im Rahmen der Gleichgewichtseinstellung. Dabei wird keine Suspension eingesetzt, sondern vorzugsweise ein Co-Emulgat aus einer lipophilen Verbindung (wobei diese bevorzugt ausgewählt ist aus Fettalkohol, Triglycerid, Paraffinöl oder Wachs) und der betreffenden Säure.

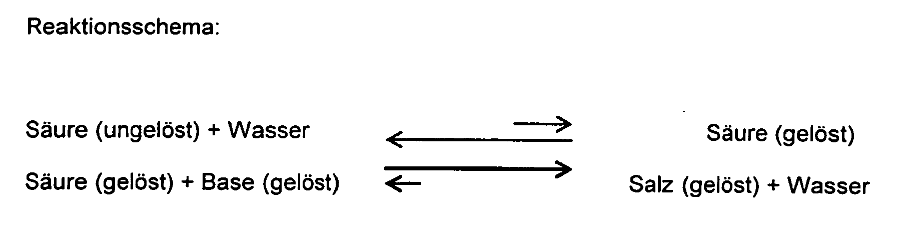

Die für die Erfindung prinzipiell geeigneten Säuren sollten vorzugsweise folgende Eigenschaften aufweisen:
- Geringe Wasserlöslichkeit (vorzugsweise unter 2 %);
- Relativ hohe Acidität;
- Niedriger Schmelzpunkt (vorzugsweise unter 150 °C);
- Kein nennenswerter Eigengeruch;
- Keine nennenswerte Beeinflussung der Standardrezeptur.

Bevorzugt ist eine Säure und/oder Base, welche bei 20 Grad Celsius in 100 ml Wasser in einer Menge von 0,01 bis 1,0g löslich ist. Besonders bevorzugt ist eine Säure und/oder Base, welche bei 20 Grad Celsius in 100 ml Wasser in einer Menge von 0,05 bis 0,5g löslich ist.

In einer bevorzugten Ausführungsform des Zweikomponenten-Mittels enthält die eine Komponente (A) als Säure eine hydrophobe, in 100 ml Wasser bei 20 Grad Celsius zu weniger als 3g lösliche Säure und die andere Komponente (B) enthält mindestens eine anorganische Base oder eine organische Base mit 1 bis 40 C-Atomen.

Die aliphatischen Monocarbonsäuren sind bei denjenigen Kettenlängen, die geruchlich akzeptabel sind, zu wenig acide, wogegen entsprechende Sulfonsäuren auch bei großen Kettenlängen zu leicht wasserlöslich sind. Überraschend besonders geeignet sind hydrophobe Dicarbonsäuren mit 6 bis 22 C-Atomen, wobei die Dicarbonsäuren mit 8 bis 16 C-Atomen sich als ideal erwiesen. Diese sind daher besonders bevorzugt. Hydrophobe Dicarbonsäuren mit 6 bis 22 C-Atomen sind einerseits geruchlos, andererseits recht acide und in einem weiten Bereich verschiedener Kettenlängen (Hydrophobizitäten) verfügbar. In diesem Bereich liegen die Dicarbonsäuren mit C₈ (Korksäure) bis C₂₂ (Docosandisäure). Sie erzeugen bei Kontakt mit unterschiedlichen Basen unter verschiedenen Bedingungen einen zeitverzögerten pH-Abfall im gewünschten Umfang. Als besonders bevorzugte Säuren sind Sebacinsäure, Dodecandisäure und Tetradecandisäure zu nennen.

Als Basen kommen vor allem anorganische Basen, insbesondere LiOH, NaOH, KOH, Ca(OH)₂, Ammoniak und Hydrazin in Betracht, während geeignete organische Basen solche mit 1 bis 30 C-Atomen sind. Beispiele für geeignete organische Basen sind primäre, sekundäre und tertiäre Alkylaminen, Alkyldiamine und Alkylolamine mit 2 bis 20 C-Atomen je Alkylrest.

Sowohl die Säure als auch die Base können jeweils in einer Konzentration von 0,01 bis 10 Gew.% bezogen auf das Gemisch der Komponenten (A) und (B) vorliegen.

Eine bevorzugte Ausführungsform des Zweikomponenten-Mittels ist dadurch gekennzeichnet, dass sich nach dem Mischen der Komponenten ein zeitlicher pH-Gradient einstellt, wobei, ausgehend vom Anfangs-pH der Mischung, der End-pH innerhalb einer Zeitspanne zwischen 2 und 100 Minuten erreicht wird.
Besonders bevorzugt ist ein Mittel, bei dem der End-pH innerhalb einer Zeitspanne zwischen 4 und 30 Minuten erreicht wird, wobei besonders bevorzugt ist, dass sich der pH vom höheren Anfangs-pH zu einem niedrigeren End-pH hin verändert.

In einer bevorzugten Ausführungsform der Erfindung kann der pH-Verlauf durch Zugabe eines oder mehrerer Indikatorfarbstoffe, die ihren Farbumschlag im Bereich der pH-Änderung haben, zu einer der Komponenten (A) oder (B) sichtbar gemacht werden. Je nach pH-Verlauf und Art des Indikators kann so ein Farbeindruck entstehen, verschwinden oder sich ändern.

Eine Erhöhung der Verzögerungszeit unter gleichzeitiger Erniedrigung des erreichbaren End-pH konnte überraschenderweise dadurch erreicht werden, dass in einer weiteren Ausführungsform eine ebenfalls wenig in Wasser lösliche Base wie z. B. Myristylamin in die alkalische Komponente (B) eingearbeitet wird. Diese kann ggf. durch Teilneutralisation mit einer geeigneten organischen oder anorganischen Säure auf den gewünschten Anfangs-pH eingestellt werden.

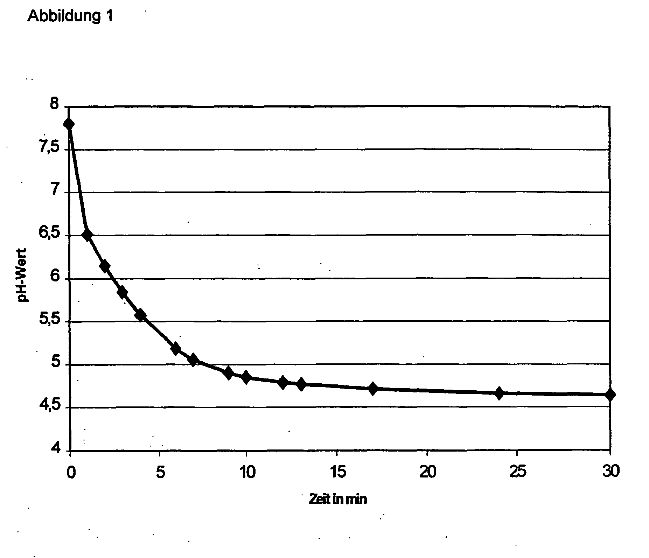

Eine weitere Ausführungsform der Erfindung besteht aus der Kombination von mittels hydrophober Dicarbonsäure teilneutralisiertem Fettamin in der Fettphase der basischen Komponente (B). Einen typischen pH-Verlauf bei Mischung gleicher Volumenteile beider Komponenten zeigt Abbildung 1.

Die Zeitdauer, Intensität, Bereichswahl und Richtung des pH-Verlaufes kann durch Auswahl und Kombination der jeweiligen Säure und Base, deren jeweiliger Einsatzkonzentration sowie deren Konzentrationsverhältnisse zueinander bestimmt und in weiten Bereichen eingestellt werden.

Die hier geschilderte Methode zum Aufbau eines zeitlichen pH-Gradienten durch retardierte Salzbildung kann genutzt werden für Produkte auf Basis von Dispersionen aller Art wie O/W- und W/O-Emulsionen, Mikroemulsionen, Suspensionen, flüssigkristallinen Phasen, Gelen, Schäumen, Aerosolen, zum Zwecke der Reinigung, Pflege, Versiegelung, Färbung oder sonstiger Behandlung von Oberflächen.

Das Zweikomponenten-Mittel ist insbesondere geeignet zur Konfektionierung als kosmetisches Mittel, vorzugsweise für die Haut-, Zahn- und Haarkosmetik. Besonders bevorzugt ist das verwendete Zweikomponenten-Mittel ein haarkosmetisches Produkt, z. B. Haarkur, Shampoo, Stylinggel, Haarfestiger, Dauerwellmittel, Fixierung, Haarbleichmittel, Haarfarbe oder Haartönung. Es enthält dann bevorzugt 1 bis 20 Gew.% einer lipophilen Verbindung, wobei diese bevorzugt ausgewählt ist aus Fettalkohol, Triglycerid, Paraffinöl oder Wachs.

Der Erfindung liegt die weitere Aufgabe zugrunde, ein Verfahren und ein Mittel zur dauerhaften Verformung von Haaren zur Verfügung zu stellen, bei dem der pH-Wert im Verlauf der Einwirkungszeit sinkt, damit die Reduktionskraft des Wellmittels während der Einwirkungszeit geschwächt und dadurch die Haarstruktur geschont wird, wobei selbstverständlich dennoch eine ausreichende Umformung der Haare erreicht werden soll.

Es wurde nunmehr überraschend gefunden, dass sich die vorstehend genannten Nachteile der Verfahren des Standes der Technik durch ein Verfahren nach Anspruch 18 vermeiden lassen.

Gegenstand der Erfindung ist daher auch ein Verfahren zur dauerhaften Verformung von Haaren nach Anspruch 18.

Bevorzugt liegt der anfängliche pH-Wert des alkalischen Dauerverformungsmittels bei 8 bis 9,5 und wird innerhalb von 10 bis 30 Minuten um 0,8 bis 2,0 Einheiten abgesenkt. Besonders bevorzugt wird der anfängliche pH-Wert des Dauerverformungsmittels innerhalb von 7 bis 20 Minuten um mindestens eine Einheit auf einen pH-Wert zwischen 6,5 bis 7,3 herabgesetzt. Die Anwendungstemperatur beträgt vorzugsweise 20 bis 45 °C, besonders bevorzugt 30 bis 40 °C.

Durch geeignete Auswahl der Säure und der Base sowie deren Konzentration lässt sich so über die Abnahme der Alkalität der Dissoziationsgrad des Reduktionsmittels und damit die Wellwirksamkeit während der Einwirkungszeit in gewünschtem Maße steuern.

Bevorzugt ist es, wenn der anfängliche pH-Wert innerhalb von 7 bis 20 Minuten um mindestens eine Einheit auf einen pH-Wert zwischen 6,5 bis 7,3 herabgesetzt wird. Besonders vorteilhaft ist es, wenn Zweikomponenten-Dauerverformungsmittel ein Dauerwellmittel ist, der pH-Wert anfänglich 8 bis 9,5 beträgt und innerhalb von 10 bis 30 Minuten um 0,8 bis 2,0 Einheiten abgesenkt wird.

In einer ebenfalls bevorzugten Ausführungsform der Erfindung ist das Zweikomponenten-Dauerverformungsmittel ein Haarentkräuselungsmittel, dessen pH-Wert anfänglich 11 bis 13 beträgt und innerhalb von 10 bis 30 Minuten um 2 bis 4 Einheiten abgesenkt wird.

Die Einwirkungszeit des Haarverformungsmittels lässt sich durch die üblicherweise bei Haarverformungsbehandlungen zum Einsatz gelangende Wärmezufuhr beschleunigen.

Als keratinreduzierender Wirkstoff kann in dem Zweikomponenten-Dauerverformungsmittel insbesondere Thioglykolsäure, Thioglykolsäureamide, Thioglycerin, Thiomilchsäure, 3-Mercaptopropionsäure, Cystein, Cysteamin, Homocystein, Alkyl- oder Acylcysteine oder die Salze dieser Verbindungen, insbesondere Ammoniumthioglykolat, verwendet werden. Der keratinreduzierende Wirkstoff ist in dem gebrauchsfertigen Mittel zur dauerhaften Haarverformung in einer Menge von 1 bis 25 Gew.-%, vorzugsweise 5 bis 15 Gew.-%, enthalten.

Selbstverständlich kann das gebrauchsfertige Mittel zur dauerhaften Verformung alle für derartige Mittel üblichen und bekannten Zusatzstoffe, z. B. Verdickungsmittel, wie beispielsweise, Bentonit, Fettsäuren, Stärke, Polyacrylsäure und deren Derivate, Cellulosederivate, Alginate, Vaseline, Paraffinöle, Netzmittel oder Emulgatoren aus den Klassen der anionischen, kationischen, amphoteren oder nichtionogenen oberflächenaktiven Substanzen, beispielsweise Fettalkoholsulfate,Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel, wie z. B. Polyethylenglykolester, Alkohole, wie z. B. ; Alkohole, wie zum Beispiel Ethanol, Propanol, Isopropanol, Polyole wie zum Beispiel Ethylenglykol, 1,2- oder 1,3-Propandiol, 1,2-, 1,3- oder 1,4-Butandiol, 1,2-, 1,3-, 1,4-oder 1,5-Pentandiol und Glycerin; Zucker wie z. B. D-Glucose, Lösungsvermittler, Stabilisatoren, Puffersubstanzen, Parfümöle, Farbstoffe sowie haarkonditionierende und haarpflegende Bestandteile, wie z. B. kationische Polymere, Lanolinderivate, Cholesterin, Pantothensäure und Betain, enthalten.

Die erwähnten Bestandteile werden in dem gebrauchsfertigen Mittel zur dauerhaften Verformung in den für solche Zwecke üblichen Mengen verwendet, z. B. die Netzmittel und Emulgatoren in Konzentrationen von insgesamt 0,2 bis 30 Gew.-%, die Alkohole in einer Menge von insgesamt 0,1 bis 20 Gew.-%, die Trübungsmittel, Parfümöle und Farbstoffe in einer Menge von jeweils 0,01 bis 1 Gew.-%, die Puffer-substanzen in einer Menge von insgesamt 0,1 bis 10 Gew.-%, Zucker, Lösungsvermittler, Stabilisatoren, sowie haarkonditionierende und haarpflegende Bestandteile in einer Menge von jeweils 0,1 bis 5 Gew.-%, während die Verdickungsmittel und Lösungsvermittler in einer Menge von insgesamt 0,5 bis 20 Gew.-% in diesem Mittel enthalten sein können.

Weiterhin können in diesem Mittel zur Wirkungssteigerung sogenannte Quell- und Penetrationsstoffe, wie z. B. Dipropylenglykolmonomethylether, 2-Pyrrolidon oder Imidazolidin-2-on, in einer Menge von 1 bis 30 Gew.-% enthalten sein.

Das gebrauchsfertige Mittel zur dauerhaften Verformung von Haaren wird erhalten durch Vermischen der Komponenten (A) und (B). Am günstigsten ist es, wenn das gebrauchsfertige Mittel unmittelbar (2 Sekunden bis 3 Minuten) vor der Anwendung durch Vermischen der zwei Komponenten hergestellt wird, wobei die Komponente (B) vorzugsweise den keratinreduzierenden Wirkstoff und die Base enthält und die Komponente (A) die Säure. Eine der beiden Komponenten kann in fester Form vorliegen, wenn die andere in flüssiger Form vorliegt; bevorzugt sind beide Komponenten flüssig oder verdickt. Das Mittel ist dabei vorteilhaft als 2-Komponentenverpackung konfektioniert.

In einer bevorzugten Ausführungsform der erfindungsgemäßen Verfahrens zur dauerhaften Haarverformung werden die Haare wie folgt behandelt: Man gibt die eine Säure enthaltende Komponente (A) vor der Anwendung zur Komponente (B), welche die Mercaptoverbindung und die Base in Lösung enthält. Die gewickelten Haare werden nun in bekannter Weise mit dem gebrauchsfertigen Verformungsmittel benetzt. Der ursprüngliche pH-Wert der Mischung von 7,5 bis 10 sinkt während der Einwirkungszeit bis auf 6,0 bis 7,5 ab. Die Einwirkungszeit ist abhängig von der Stärke der gewünschten Krause und von der Behandlungstemperatur.

Nach einer für die dauerhafte Verformung des Haares ausreichenden Einwirkungszeit, welche je nach Haarbeschaffenheit, der Verformungswirksamkeit des Verformungsmittels sowie in Abhängigkeit von der Anwendungstemperatur 5 bis 30 Minuten (10 bis 30 Minuten ohne Wärmeeinwirkung oder 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignete Nachbehandlungsmittel verwendet werden. Beispiele für in solchen Nachbehandlungsmitteln verwendbare Oxidationsmittel sind Kalium- und Natriumbromat, Natriumperborat, Harnstoff- und Wasserstoffperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wässrigen Nachbehandlungsmittel in einer Konzentration von 0,5 bis 10 Gew.-% vor. Das Mittel für die oxidative Nachbehandlung kann selbstverständlich weitere Stoffe, wie z. B. Netzmittel, Pflegestoffe wie kationaktive Polymere, schwache Säuren, Puffersubstanzen oder Peroxidstabilisatoren, enthalten und in Form einer wässrigen Lösung, einer Emulsion sowie in verdickter Form auf wässriger Basis, insbesondere als Creme, Gel oder Paste, vorliegen. Diese üblichen Zusätze können insbesondere in einer Menge von 0,1 bis 10 Gew.-% in dem Nachbehandlungsmittel enthalten sein.

Anschließend werden die Wickler entfernt. Falls erforderlich, kann das abgewickelte Haar nun nochmals oxidativ nachbehandelt werden. Dann wird das Haar mit Wasser gespült, gegebenenfalls zur Wasserwelle gelegt und schließlich getrocknet.

Das Mittel bzw. das Verfahren bewirkt eine elastische, dauerhafte und gleichmäßige Umformung vom Haaransatz bis zur Haarspitze, ohne allergische oder sensibilisierende Reaktionen hervorzurufen unter weitgehender Schonung der Haarstruktur vor Schädigung.

Ist das Mittel eine Zweikomponenten-Haarkur, so werden vorzugsweise zwei kationische Haarpflegekuren hergestellt, die unmittelbar vor der Anwendung miteinander innig vermischt werden: Eine saure Kur (**Kur S**), die in der Fettalkoholphase zusätzlich eine Carbonsäure enthält (dabei wird auf die sonst übliche Ansäuerung verzichtet und der pH liegt bei der Kur S bereits zwischen 3 und 4) und eine basisch eingestellte Kur (**Kur B**). Bei Säuren mit höherem Schmelzpunkt, wie Sebacin- oder Korksäure, muss im Glycerin- oder Ölbad bei ca. 120 °C aufgeschmolzen werden. Der zeitliche pH-Verlauf kann entweder über pH-Messung überwacht werden und/oder mittels geeigneter Indikatorfarbstoffe sichtbar gemacht werden. Einen typischen pH-Verlauf bei mischungen gleicher Volumina von Kur S und Kur B zeigt Abbildung 1.

### Beispiel 1 Zweikomponenten-Haarkur mit Farbwechsel am Ende der empfohlenen Einwirkungszeit

| **Rohstoffe** | **saure Komponente A** | **basische Komponente B** |
|---|---|---|
| | **(Kur S)** | **(Kur B)** |
| Sebacinsäure | 2,0 g | 0,7 g |
| Myristylamin | - | 2,0 g |
| Bromthymolblau (pH-Indikator) | - | 0,2 g |
| Cetylstearylalkohol (Lanette^{®} O) | 4,0 g | 4,0 g |
| Cetyltrimethylammoniumchlorid | 1,0 g | 1,0 g |
| Wasser | ad 100,0 g | ad 100,0 g |
| pH-Wert | 3,0 | 9,5 |

### Herstellung:

**Kur S** (Komponente A): Der Fettalkohol wird gemeinsam mit der hydrophoben Säure bei 90 °C im Wasserbad unter Rühren geschmolzen, bis sich eine klare Phase ergibt. Die Wasserphase, bestehend aus Cetyltrimethylammoniumchlorid und Wasser, wird auf 85 °C erhitzt. Diese wird in die flüssige Fettphase bei 85 °C unter Rühren einemulgiert. In einem kalten Wasserbad wird die entstandene Emulsion ebenfalls unter Rühren auf Raumtemperatur abgekühlt.

**Kur B** (Komponente B): Der Fettalkohol wird gemeinsam mit der hydrophoben Säure, dem Fettamin und dem Indikatorfarbstoff bei 90 °C im Wasserbad unter Rühren geschmolzen, bis sich eine klare Phase ergibt. Die Wasserphase, bestehend aus Cetyltrimethylammoniumchlorid und Wasser, wird auf 85 °C erhitzt. Diese wird in die flüssige Fettphase bei 85 °C unter Rühren einemulgiert. In einem kalten Wasserbad wird die entstandene Emulsion ebenfalls unter Rühren auf Raumtemperatur abgekühlt.

**Anwendung:** Unmittelbar vor dem Gebrauch werden gleiche Volumina von Kur S und Kur B innig miteinander vermischt. Im Endprodukt sollte, bei Verwendung einer Zweikomponentenverpackung, zumindest eine Vormischung (z. B. wie bei einer Streifen-Zahncreme) erfolgen. Beim Einarbeiten ins Haar wird dann die endgültige Durchmischung vollzogen.

Der Anfangs-pH der Mischung liegt bei 7,8. Die vorliegende Zweikomponenten-Kur ist darauf eingerichtet, dass bei Applikation gleicher Volumina der beiden Komponenten nach ca. 10 Minuten an allen Stellen der Farbendwert erreicht worden ist. Abweichungen von diesem Mischungsverhältnis zugunsten der sauren Komponente A oder zugunsten der basischen Komponente B führen demgegenüber zu einer Beschleunigung bzw. Verzögerung des Farbumschlages.

Die Kur hat unmittelbar nach dem Vermischen eine kräftig blaue Farbe, die sich im Laufe von 8 bis 12 Minuten immer mehr zu einem blassen Gelbton verändert.

### Beispiel 2 Dauerwellmittel mit pH-Absenkung

| **Rohstoffe** | **saure Komponente A** | **basische Komponente B** |
|---|---|---|
| Sebacinsäure | 1,0 g | - |
| Hexadecylamin | - | 1,0 g |
| Cetylstearylalkohol | 2,0 g | 2,0 g |
| (Lanette O) | | |
| ethoxylierter Cetylsteary- | 0,5 g | 0,5 g |
| lalkohol (25 EO) | | |
| Thioglykolsäure | 10,5 g | 10,5 g |
| Ammoniak 25%ig | 5,7 g | 10,6 g |
| Wasser | ad 100,0 g | ad 100,0 g |
| **pH-Wert:** | **3,5** | **9,1** |

Unmittelbar vor der Anwendung werden beide Komponenten A und B im Verhältnis 1 : 1 vermischt und anschließend das gebrauchsfertige Dauerwellmittel auf das Haar aufgebracht. Man erhält für das Gemisch einen pH-Wert von 8,5 der sich im Verlauf der Einwirkungszeit von 15 Minuten bis auf einen End-pH-Wert von 7,0 verringert.

### Beispiel 3 Cremehaarfarbe Kupferrot

| **Rohstoff** | **saure Komponente A** | **basische Komponente B** |
|---|---|---|
| Tetradecandisäure | 2,00 g | - |
| Ammoniak - | - | 2,00 g |
| ethoxylierter Laurylalkohol, (3 , EO) | 4,00 g | 4,00 g |
| C₁₆₋₁₈-Fettalkohol | 8,00 g | 8,00 g |
| Komplexbildner | - | 0,20 g |
| Natriumsulfit | - | 1,00 g |
| Parfümöl | 0,50 g | 0,50 g |
| p-Aminophenol | - | 0,10 g |
| p-Toluylendiamin : | | 0,20 g |
| o-Nitro-p-phenylendiamin | - | 0,10 g |
| p-Nitro-o-aminophenol | - | 0,05 g |
| Resorcin | - | 0,05 g |
| Wasserstoffperoxid | 6,00 g | - |
| Wasser | ad 100,00 g | ad 100,00 g |
| **pH Wert:** | **ca. 3,0** | **ca. 10,0** |

Unmittelbar vor der Anwendung werden beide Komponenten A und B vermischt und anschließend die gebrauchsfertige Cremehaarfarbe auf das Haar aufgebracht. Man erhält für das Gemisch einen pH-Wert von 9,5 der im Lauf der Einwirkungszeit von 12 Minuten allmählich in den neutralen pH-Bereich absinkt.

## Patentansprüche

1. Verfahren zur Behandlung von Haaren, bei dem man unmittelbar vor der Anwendung eine Komponente (A) eines Zweikomponenten-Mittels, die mindestens eine Säure enthält mit einer Komponente (B) des Zweikomponenten-Mittels, die mindestens eine Base enthält, vermischt, wobei sich nach dem Vermischen der Komponenten der pH-Wert allmählich innerhalb von 1 bis 60 Minuten selbsttätig verändert, das Haar mit dem gebrauchsfertigen Mittel behandelt und nach der Einwirkungszeit aus dem Haar ausspült, **dadurch gekennzeichnet, dass** die Säure und/oder die Base in 100 ml Wasser bei 20 Grad Celsius zu weniger als 3g löslich ist.

2. Verfahren nach Anspruch 1, **dadurch gekennzeichnet, dass** die Säure und/oder die Base bei 20 Grad Celsius in 100 ml Wasser in einer Menge von 0,01 bis 0,5g löslich ist.

3. Verfahren nach Anspruch einem der Ansprüche 1 oder 2, **dadurch gekennzeichnet, dass** die Komponente (A) als Säure eine hydrophobe, in 100 ml Wasser bei 20 Grad Celsius zu weniger als 3g lösliche Säure enthält und die Komponente (B) mindestens eine anorganische Base oder eine organische Base mit 1 bis 40 C-Atomen enthält.

4. Verfahren nach einem der Ansprüche 1 bis 3, **dadurch gekennzeichnet, dass** die hydrophobe Säure eine Dicarbonsäure mit 6 bis 22 C-Atomen ist.

5. Verfahren nach Anspruch 4, **dadurch gekennzeichnet, dass** die hydrophobe Säure eine Dicarbonsäure mit 8 bis 16 C-Atomen ist.

6. Verfahren nach einem der Ansprüche 1 bis 5, **dadurch gekennzeichnet, dass** die hydrophobe Säure ausgewählt ist aus Sebacinsäure, Dodecandisäure und Tetradecandisäure.

7. Verfahren nach einem der Ansprüche 1 bis 6, **dadurch gekennzeichnet, dass** die Base eine anorganische Base ist und ausgewählt ist aus LiOH, NaOH, KOH, Ca(OH)₂, Ammoniak und Hydrazin.

8. Verfahren nach einem der Ansprüche 1 bis 6, **dadurch gekennzeichnet, dass** die Base eine organische Base mit 1 bis 20 C-Atomen ist.

9. Verfahren nach Anspruch 8, **dadurch gekennzeichnet, dass** die organische Base ausgewählt ist aus Alkylaminen und Alkylolaminen.

10. Verfahren nach einem der Ansprüche 1 bis 9, **dadurch gekennzeichnet, dass** die Säure und die Base jeweils in einer Konzentration von 0,01 bis 10 Gew.% bezogen auf das Gemisch der Komponenten (A) und (B) vorliegen.

11. Verfahren gemäß einem der Ansprüche 1 bis 10, **dadurch gekennzeichnet, dass** sich nach dem Mischen der Komponenten ein zeitlicher pH-Gradient einstellt, wobei, ausgehend vom Anfangs-pH der Mischung, der End-pH innerhalb einer Zeitspanne zwischen 2 und 100 Minuten erreicht wird.

12. Verfahren nach Anspruch 11, **dadurch gekennzeichnet, dass**, der End-pH innerhalb einer Zeitspanne zwischen 4 und 30 Minuten erreicht wird.

13. Verfahren nach Anspruch 12, **dadurch gekennzeichnet, dass** sich der pH vom höheren Anfangs-pH zu einem niedrigeren End-pH hin verändert.

14. Verfahren nach einem der Ansprüche 1 bis 13, **dadurch gekennzeichnet, dass** mindestens eine der Komponeneten (A) oder (B) einen pH-Indikator enthält, der seinen Farbumschag im Bereich der pH-Änderung hat.

15. Verfahren nach Anspruch 1, **dadurch gekennzeichnet, dass** das verwendete Mittel ein Haarpflegemittel oder Haardauerverformungsmittel ist.

16. Verfahren nach einem der Ansprüche 1 bis 15, **dadurch gekennzeichnet, dass** das verwendete Mittel 1 bis 20 Gew.% einer lipophilen Verbindung enthält.

17. Verfahren nach Anspruch 16, **dadurch gekennzeichnet, dass** die lipophilen Verbindung ausgewählt ist aus Fettalkohol, Triglycerid, Paraffinöl oder Wachs.

18. Verfahren zur dauerhaften Verformung von Haaren, bei dem man unmittelbar vor der Anwendung die Komponente (B) eines Zweikomponenten-Dauerverformungsmittels mit einem Gehalt an einem keratinreduzierenden Wirkstoff und einer Base mit einem anfänglichen pH-Wert von 7,5 bis 10 mit der zweiten Komponente (A), enthaltend eine Säure, zum gebrauchsfertigen Dauerverformungsmittel vermischt, wobei sich nach dem Vermischen der Komponenten der pH-Wert allmählich innerhalb von 1 bis 60 Minuten selbsttätig verändert, das Haar bevor und/oder nachdem man es in der gewünschten Form festhält, mit dem gebrauchsfertigen Mittel behandelt, nach der Einwirkungszeit das Haar mit Wasser spült, oxidativ nachbehandelt, erneut mit Wasser spült, gegebenenfalls zur Wasserwelle legt, und sodann trocknet, **dadurch gekennzeichnet, dass** die Säure und/oder die Base in 100 ml Wasser bei 20 Grad Celsius zu weniger als 3g löslich ist.

19. Verfahren nach einem der Ansprüche 1 oder 18, **dadurch gekennzeichnet, dass** der anfängliche pH-Wert des gebrauchsfertigen Zweikomponenten-Mittels innerhalb von 7 bis 20 Minuten um mindestens eine Einheit auf einen pH-Wert zwischen 6,5 bis 7,3 herabgesetzt wird.

20. Verfahren nach Anspruch 18, **dadurch gekennzeichnet, dass** das Zweikomponenten-Dauerverformungsmittel ein Dauerwellmittel ist, der pH-Wert anfänglich 8 bis 9,5 beträgt und innerhalb von 10 bis 30 Minuten um 0,8 bis 2,0 Einheiten abgesenkt wird.

21. Verfahren nach Anspruch 18, **dadurch gekennzeichnet, dass** das Zweikomponenten-Dauerverformungsmittel ein Haarentkräuselungsmittel ist, der pH-Wert anfänglich 11 bis 13 beträgt und innerhalb von 10 bis 30 Minuten um 2 bis 4 Einheiten abgesenkt wird.

## Claims

1. Process for treating hair in which, directly prior to use, a component (A) of a two-component agent which comprises at least one acid is mixed with a component (B) of the two-component agent which comprises at least one base, where, after mixing the components, the pH gradually changes automatically within from 1 to 60 minutes, the hair is treated with the ready-to-use agent and, after the contact time, is rinsed out of the hair, **characterized in that** the acid and/or the base is soluble in 100 ml of water at 20 degrees Celsius to less than 3 g.

2. Process according to Claim 1, **characterized in that** the acid and/or the base is soluble at 20 degrees Celsius in 100 ml of water in an amount of from 0.01 to 0.5 g.

3. Process according to either of Claims 1 and 2, **characterized in that** component (A) comprises, as acid, a hydrophobic acid soluble in 100 ml of water at 20 degrees Celsius to less than 3 g, and component (B) comprises at least one inorganic base or an organic base having 1 to 40 carbon atoms.

4. Process according to one of Claims 1 to 3, **characterized in that** the hydrophobic acid is a dicarboxylic acid having 6 to 22 carbon atoms.

5. Process according to Claim 4, **characterized in that** the hydrophobic acid is a dicarboxylic acid having 8 to 16 carbon atoms.

6. Process according to one of Claims 1 to 5, **characterized in that** the hydrophobic acid is chosen from sebacic acid, dodecanedioic acid and tetradecanedioic acid.

7. Process according to one of Claims 1 to 6, **characterized in that** the base is an inorganic base and is chosen from LiOH, NaOH, KOH, Ca(OH)₂, ammonia and hydrazine.

8. Process according to one of Claims 1 to 6, **characterized in that** the base is an organic base having 1 to 20 carbon atoms.

9. Process according to Claim 8, **characterized in that** the organic base is chosen from alkylamines and alkylolamines.

10. Process according to one of Claims 1 to 9, **characterized in that** the acid and the base are in each case present in a concentration of from 0.01 to 10% by weight, based on the mixture of components (A) and (B).

11. Process according to one of Claims 1 to 10, **characterized in that**, after mixing the components, a temporal pH gradient is established where, starting from the initial pH of the mixture, the end pH is reached within a period between 2 and 100 minutes.

12. Process according to Claim 11, **characterized in that** the end pH is reached within a period between 4 and 30 minutes.

13. Process according to Claim 12, **characterized in that** the pH changes from the higher starting pH to a lower end pH.

14. Process according to one of Claims 1 to 13, **characterized in that** at least one of components (A) or (B) comprises a pH indicator which has its colour change in the range of the pH change.

15. Process according to Claim 1, **characterized in that** the agent used is a haircare agent or hair permanent shaping agent.

16. Process according to one of Claims 1 to 15, **characterized in that** the agent used comprises 1 to 20% by weight of a lipophilic compound.

17. Process according to Claim 16, **characterized in that** the lipophilic compound is chosen from fatty alcohol, triglyceride, paraffin oil or wax.

18. Process for perming hair in which, directly prior to use, component (B) of a two-component permanent shaping agent with a content of a keratin-reducing active ingredient and a base with an initial pH of from 7.5 to 10 is mixed with the second component (A) comprising an acid to give the ready-to-use permanent shaping agent, while, after mixing the components, the pH gradually changes automatically within 1 to 60 minutes, the hair is treated with the ready-to-use agent before and/or after it is held in the desired shape, after the contact time the hair is rinsed with water, oxidatively after-treated, rinsed with water again, optionally set and then dried, **characterized in that** the acid and/or the base is soluble in 100 ml of water at 20 degrees Celsius to less than 3 g.

19. Process according to one of Claims 1 or 18, **characterized in that** the initial pH of the ready-to-use two-component agent is reduced within from 7 to 20 minutes by at least one unit to a pH between 6.5 and 7.3.

20. Process according to Claim 18, **characterized in that** the two-component permanent shaping agent is a permanent waving agent, the pH is initially 8 to 9.5 and is reduced within from 10 to 30 minutes by 0.8 to 2.0 units.

21. Process according to Claim 18, **characterized in that** the two-component permanent shaping agent is a hair straightening agent, the pH is initially 11 to 13 and is reduced within from 10 to 30 minutes by 2 to 4 units.

## Revendications

1. Procédé pour le traitement des cheveux, dans lequel on mélange immédiatement avant l'emploi un composant (A) d'une composition bicomposant, qui contient au moins un acide, avec un composant (B) de la composition bicomposant, qui contient au moins une base, le pH se modifiant de lui-même progressivement en l'espace de 1 à 60 minutes après le mélange des composants, on traite les cheveux par la composition prête à l'emploi et après le temps d'action on l'élimine par rinçage des cheveux, **caractérisé en ce que** l'acide et/ou la base est(sont) soluble(s) à raison de moins de 3 g dans 100 ml d'eau à 20 °C.

2. Procédé selon la revendication 1, **caractérisé en ce que** l'acide et/ou la base est(sont) soluble(s) en une quantité de 0,01 à 0,5 g dans 100 ml d'eau à 20 °C.

3. Procédé selon la revendication 1 ou 2, **caractérisé en ce que** le composant (A) contient en tant qu'acide un acide hydrophobe soluble à raison de moins de 3 g dans 100 ml d'eau à 20 °C et le composant (B) contient au moins une base minérale ou une base organique ayant de 1 à 40 atomes de carbone.

4. Procédé selon l'une quelconque des revendications 1 à 3, **caractérisé en ce que** l'acide hydrophobe est un acide dicarboxylique ayant de 6 à 22 atomes de carbone.

5. Procédé selon la revendication 4, **caractérisé en ce que** l'acide hydrophobe est un acide dicarboxylique ayant de 8 à 16 atomes de carbone.

6. Procédé selon l'une quelconque des revendications 1 à 5, **caractérisé en ce que** l'acide hydrophobe est choisi parmi l'acide sébacique, l'acide dodécanedioïque et l'acide tétradécanedioïque.

7. Procédé selon l'une quelconque des revendications 1 à 6, **caractérisé en ce que** la base est une base minérale et est choisie parmi LiOH, NaOH, KOH, Ca(OH)₂, l'ammoniac et l'hydrazine.

8. Procédé selon l'une quelconque des revendications 1 à 6, **caractérisé en ce que** la base est une base organique ayant de 1 à 20 atomes de carbone.

9. Procédé selon la revendication 8, **caractérisé en ce que** la base organique est choisie parmi des alkylamines et alkylolamines.

10. Procédé selon l'une quelconque des revendications 1 à 9, **caractérisé en ce que** l'acide et la base sont présents chacun à une concentration de 0,01 à 10 % en poids, par rapport au mélange des composants (A) et (B).

11. Procédé selon l'une quelconque des revendications 1 à 10, **caractérisé en ce qu'**après le mélange des composants il s'établit un gradient du pH dans le temps, de sorte qu'à partir du pH initial du mélange, le pH final est atteint en un espace de temps compris entre 2 et 100 minutes.

12. Procédé selon la revendication 11, **caractérisé en ce que** le pH final est atteint en un espace de temps compris entre 4 et 30 minutes.

13. Procédé selon la revendication 12, **caractérisé en ce que** le pH se modifie du pH initial supérieur à un pH final plus bas.

14. Procédé selon l'une quelconque des revendications 1 à 13, **caractérisé en ce qu'**au moins l'un des composants (A) et (B) contient un indicateur de pH, qui présente son virage de couleur dans l'intervalle de la modification du pH.

15. Procédé selon la revendication 1, **caractérisé en ce que** la composition utilisée est un produit de soin capillaire ou un produit de mise en forme permanente des cheveux.

16. Procédé selon l'une quelconque des revendications 1 à 15, **caractérisé en ce que** la composition utilisée contient de 1 à 20 % en poids d'un composé lipophile.

17. Procédé selon la revendication 16, **caractérisé en ce que** le composé lipophile est choisi parmi un alcool gras, un triglycéride, une huile de paraffine et une cire.

18. Procédé pour la mise en forme permanente des cheveux, dans lequel on mélange immédiatement avant l'emploi le composant (B) d'une composition bicomposant de mise en forme permanente, ayant une teneur en une substance active réduisant la kératine et en une base, ayant un pH initial de 7,5 à 10, avec le second composant (A) contenant un acide, pour l'obtention de la composition de mise en forme permanente prête à l'emploi, de sorte qu'après le mélange des composants le pH se modifie progressivement de lui-même en l'espace de 1 à 60 minutes, on traite les cheveux par la composition prête à l'emploi, avant et/ou après les avoir maintenus en la forme désirée, après le temps d'action on rince les cheveux à l'eau, on les soumet à un après-traitement oxydant, on les rince de nouveau à l'eau, éventuellement on les met en plis et ensuite on les sèche, **caractérisé en ce que** l'acide et/ou la base est(sont) soluble(s) à raison de moins de 3 g dans 100 ml d'eau à 20 °C.

19. Procédé selon la revendication 1 ou 18, **caractérisé en ce que** le pH initial de la composition bicomposant prête à l'emploi est abaissé en l'espace de 7 à 20 minutes d'au moins une unité, à un pH compris entre 6,5 et 7,3.

20. Procédé selon la revendication 18, **caractérisé en ce que** la composition bicomposant de mise en forme permanente est une composition d'ondulation permanente, le pH est initialement de 8 à 9,5 et est abaissé de 0,8 à 2,0 unités en l'espace de 10 à 30 minutes.

21. Procédé selon la revendication 18, **caractérisé en ce que** la composition bicomposant de mise en forme permanente est une composition de défrisage des cheveux, le pH est initialement de 11 à 13 et est abaissé de 2 à 4 unités en l'espace de 10 à 30 minutes.
